# EUROPEAN PATENT APPLICATION

(11) **EP 1 193 312 A1**
(43) Date of publication of application: **03.04.2002**
(21) Application number: 00931567.2
(22) Date of filing: 25.05.2000
(51) Int. Cl.: C12N 15/11, C12Q 1/68, C12N 1/20

(54) **NOVEL PSYCHROTROPHIC BACTERIUM AND DNA PROBE FOR DETECTING THE BACTERIUM**

(30) Priority: 25.05.1999 JP 14534299; 30.03.2000 WO PCT/JP00/02045
(71) Applicant: National Institute of Advanced Industrial Science and Technology, Tokyo 100-8921 (JP)
(72) Inventor: MARUYAMA, Akihiko, Tsukuba-shi, Ibaraki 305-0031 (JP); KITAMURA, Keiko, Tsukuba-shi, Ibaraki 305-0061 (JP); KURANE, Ryuichiro, Matsudo-shi, Chiba 270-0031 (JP)
(74) Representative: Maschio, Antonio
(86) International application number: JP0003372
(87) International publication number: WO0071705

(57) **Abstract**

The present invention is aimed to provide a technique for species-specifically detecting a microorganism naturally inhabiting in the deep sea or an analog thereof, based on the characteristics of its genetic information. The present invention provides a 16S rDNA which has the base sequence of SEQ ID NO:1, an oligonucleotide probe which comprises part of the base sequence of SEQ ID NO:1 and a method for specifically detecting or identifying a bacterium belonging to *Psychrobacter pacificensis* using the probe. The oligonucleotide probe of the present invention can detect *Psychrobacter pacificensis* at a molecular or cell level at high sensitivity and high accuracy as a useful indicator organism in monitoring of the circulation of deep-layer seawater.

## Description

### Technical Field

The present invention relates to a technique for monitoring the circulation and upwelling of deep-sea water using a deep-sea microorganism as an indicator, and particularly to a technique for species-specifically detecting a bacterium selected from a group consisting of *Psychrobacter pacificensis*, *Psychrobacter glacincola* and analogs thereof, as well as a technique for species-specifically detecting a bacterium belonging to *Psychrobacter pacificensis*. The present invention also relates to a deep-sea microorganism which can be used as an indicator for monitoring the circulation and upwelling of deep-sea water, and more particularly to a bacterium belonging to *Psychrobacter pacificensis.*

### Background of the Invention

It is thought that waters in the deep-sea near the Japan islands are supplied by a great ocean current which originally starts from the high-density sea water in the depth of the sea near Greenland and via the Antarctic region where other high-density sea water merges with the current, flows to northern parts of the Pacific Ocean including the Japan Trench. Such deep-sea water contains plenty of nutritive salts and exhibits a high productivity of organisms in its upwelling area. Accordingly, the industrial applicability of such deep-sea waters is now explored.

Further, deep-sea fish, which had not been utilized to date, are now beginning to be used as food or feed.

Moreover, it is suggested in connection with problems of global or local environmental pollution, that CO₂, radioactive waste or industrial waste resulting from human activities be disposed into the deep-sea zone near Japan.

However, because there is little knowledge concerning deep-sea waters or zones, it is difficult to evaluate what effect deep-sea water has on the activities of organisms inhabiting in epipelagic zones, or what effect the disposal of, for example, CO₂, radioactive waste or industrial waste into the deep-sea would have on the activities of organisms in the deep-sea. Further, no indicator organism has been reported which may provide useful information on the global ocean current of deep-layer sea water.

### Summary of the Invention

The object of the present invention is to provide a technique for evaluating the biological safety of artificial use of deep-sea water or deep-sea zones, and particularly a technique for species-specifically detecting a microorganism naturally inhabiting in the deep sea or an analog thereof, based on the characteristics of its genetic information.

The present inventors developed an oligonucleotide probe which enables species-specific detection of a novel psychrotrophic bacterium species isolated from the deep-sea water of the Japan Trench, at a molecular or cell level based on base sequence information of 16S rRNA derived from the microorganism, thereby completing the present invention.

In summary, the present invention provides a 16S rDNA which has the base sequence of SEQ ID NO:1.

The present invention also provides an oligonucleotide probe which comprises part of the base sequence of SEQ ID NO:1. The oligonucleotide probe may be either an RNA or DNA probe. One example of said part of the base sequence of SEQ ID NO:1 is the base sequence of SEQ ID NO:2. Such a probe can be used to detect or identify a bacterium selected from the group consisting of *Psychrobacter pacificensis, Psychrobacter glacincola* and analogs thereof. It may also be used to specifically detect or identify a bacterium belonging to *Psychrobacter pacificensis*.

The present invention also provides a method for detecting or identifying a bacterium selected from the group consisting of *Psychrobacter pacificensis*, *Psychrobacter glacincola* and analogs thereof, using an oligonucleotide probe comprising part of the base sequence of SEQ ID NO:1.

Further, the present invention provides a method for specifically detecting or identifying a bacterium belonging to *Psychrobacter pacificensis* by using an oligonucleotide probe comprising part of the base sequence of SEQ ID NO:1.

Further, the present invention provides *Psychrobacter pacificensis* which is aerobic, gram-negative, nonmotile, colorless, non-sporulating and oxidase-positive. One example of *Psychrobacter pacificensis* strain is *Psychrobacter pacificensis* NIBH P2K6 (FERM BP-7106).

Contents of Japanese Patent Application No. 11-145342 and International Patent Application No. PCT/JP00/02045 are incorporated herein by reference.

### Description of the Sequence List

SEQ ID NO:2 : synthetic DNA (probe Psypac469-487)
SEQ ID NO:3 : synthetic DNA (primer 359f)
SEQ ID NO:4 : synthetic DNA (primer 803r)
SEQ ID NO:5 : synthetic DNA (primer 821f)
SEQ ID NO:6 : synthetic DNA (primer 1104r)
SEQ ID NO:7 : synthetic DNA (primer 1111f)
SEQ ID NO:8 : synthetic DNA (probe Eub338-355)
SEQ ID NO:9 : synthetic DNA (probe Cont)
SEQ ID NO:10 : synthetic DNA (probe Univ1390-1407)

### Best Modes for Carrying out the Invention

The newly-discovered microorganism species according to the present invention, *Psychrobacter pacificensis*, is a heterotrophic microorganism which predominantly appears under cold culture conditions of 1 atm, at 4°C, that was isolated from the seawater of the Japan Trench in the offing of Hachijo island, Japan at a depth of 6,000 meters. Six strains of *Psychrobacter pacificensis,* NIBH P2J2, NIBH P2J3, NIBH P2J13, NIBH P2K6, NIBH P2K17 and NIBH P2K18 have been isolated by the present inventors. Although the microorganism species had initially been named *"Psychrobacter pacificus"* by the present inventors, it was later renamed *"Psychrobacter pacificensis"* and registered as a new species (Maruyama et al., Phylogenetic analysis of psychrophilic bacteria isolated from the Japan Trench, including a description of the deep-sea species *Psychrobacter pacificensis* sp. nov., Inter. J. Syst. Evol. Microbiol., 50 (2000) 835-846). Accordingly, both the above-described *Psychrobacter pacificus* and *Psychrobacter pacificensis* refer to the same microorganism group.

Among the above-described strains, phylogenetic characteristics of NIBH P2J3, NIBH P2K6 and NIBH P2K18 are shown in Tables 1-3 below. Tables 1-3 also include other strains isolated simultaneously with, and analogs of, the above-described strains, together with their phylogenetic characteristics.

**Table 1**

| Motility and Extracellular Organ of Psychrotrophic Bacteria Isolated from Surface and Deep Seawaters of the Japan Trench | | | | |
|---|---|---|---|---|
| Strain | Motility test¹ (microscopic) | Motility test² (agar plate) | Extracellular organ³ | Phylogenetic location |
| Surface seawater | | | | |
| P1H8 | - | - | Flagella* | *Halomonadaceae* |
| P1H13 | - | - | None | *Halomonadaceae* |
| P1H14 | - | - | None | *Halomonadaceae* |
| P1H22 | + | + | Flagella | *Halomonadaceae* |
| P1H25 | + | + | Flagella | *Halomonadaceae* |
| | | | | |

| Deep seawater | | | | |
|---|---|---|---|---|
| P2J2 | -/w | - | Fimbriae | *Moraxellaceae* |
| P2J3 | -/w | - | Fimbriae | *Moraxellaceae* |
| P2J13 | -/w | - | Fimbriae | *Moraxellaceae* |
| P2K6 | -/w | - | Fimbriae | *Moraxellaceae* |
| P2K17 | -/w | - | Fimbriae | *Moraxellaceae* |
| P2K18 | -/w | - | Fimbriae | *Moraxellaceae* |

| | | | | |
|---|---|---|---|---|
| 1: By optical microscopy using Nomarski optics. 2: On a semisolid agar medium with nutrient gradient. 3: By electron microscopy:- (negative); Flagella* (frequent adhesion of flagella was observed); w (weak twitch). | | | | |

**Table 2**

| Characteristics of Phenotypes and GC Contents of *Psychrobacter pacificensis* Strains and Their Analogs | | | | | | | | | |
|---|---|---|---|---|---|---|---|---|---|
| Characteristics ^{a)} | *Psychrobacter pacificensis* NIBH strain no. | | | | *Psychrobacter immobilis* ^{(b} | *Psychrobacter urativorans* ^{(b} | *Psychrobacter frigidicola* ^{(b} | *Psychrobacter phenylpyruvicus* ACAM 535^{(b} | *Psychrobacter glacincola* ACAM 483 ^{c)} |
| | P2J3 | P2K6 | P2K18 | Summary | (Phenon 1) | (Phenon 2) | (Phenon 3) | | |
| Urease activity | + | + | + | + | v+ | v+ | - | + | v- |
| Phenylalanine deaminase | - | - | - | - | + | - | + | + | - |
| Tryptophan deaminase | - | - | - | - | v- | - | + | - | - |
| Nitrate reduction | - | - | - | - | v- | v- | - | - | v+ |

| Growth in NaCl (%): | | | | | | | | | |
|---|---|---|---|---|---|---|---|---|---|
| 0 | w | - | - | - | + | + | + | ? | + |
| 1 | + | - | - | -v | + | + | + | + | + |
| 3 | + | + | + | + | + | + | + | + | + |
| 3 | + | + | + | + | + | + | + | + | + |
| 8 | - | - | - | - | + | + | + | + | + |

| Growth at (°C): | | | | | | | | | |
|---|---|---|---|---|---|---|---|---|---|
| 30 | + | + | + | + | + | - | - | + | - |
| 35 | + | w | + | + | v+ | - | - | + | - |
| 40 | - | - | - | - | | | | | |

| Acid production from: | | | | | | | | | |
|---|---|---|---|---|---|---|---|---|---|
| Glucose | + | w | + | + | | | | | |
| Xylose | + | w | + | + | + | - | - | - | - |
| Arabinose | + | + | + | + | + | - | - | - | - |
| *Others^{(d} | - | - | - | - | - | - | - | - | - |
| PNPG tes^{(d} | - | - | - | - | | | | | |

| Use as sole carbon and energy sources: | | | | | | | | | |
|---|---|---|---|---|---|---|---|---|---|
| Acetate | + | - | - | -v | + | v+ | + | + | + |
| L-alanine | + | - | - | -v | + | - | - | + | v+ |
| p-hydroxy-benzoate | - | - | - | - | - | v- | - | - | ? |
| 3-hydroxy-butyrate | + | - | + | +v | + | + | - | + | v+ |
| Citrate | - | - | - | - | v- | - | - | + | v+ |
| Gluconate^{(d} | - | - | - | - | v- | - | - | - | - |
| L-histidine | + | + | + | + | + | - | - | - | v+ |
| Lactate | + | - | + | +v | +(DL) | v+(DL) | -(DL) | + (DL) | v+(DL) |
| DL-malate^{(d} | + | + | + | + | +(L) | v+(L) | +(L) | +(L) | -(L) |
| Malonate | - | + | - | -v | - | - | - | - | - |
| Propionate | - | - | - | - | v+ | - | - | + | + |
| L-serine | - | - | - | - | - | v- | - | - | - |
| Suberate | + | - | - | -v | v- | - | + | - | ? |
| n-valerate | - | - | - | - | + | v+ | + | + | + |
| **Others | - | - | - | - | | | | | |
| DNA G+C content (mol %) | 44 | 44 | 45 | 44-45 | 44-47 | 44-46 | 41-42 | 43 | 43-44 |

| | | | | | | | | | |
|---|---|---|---|---|---|---|---|---|---|
| a) All of the species and strains were proved to be positive for oxidase, catalase, culture at 4-15°C, resistance against 6.5% NaCl, as well as for the use of L-proline as sole carbon and energy sources. b) Data from Bowman et al., (1996) Int. J. Syst. Bacteriol. 46:841-848. c) Data from Bowman et al., (1997) System. Appl. Microbiol. 20:209-215. d) Determined by API 20 NE test. Compound availability was estimated by using API ID 32 GN test. PNPG is a test for β-galactosidase using para-nitrophenyl-(β)-D-galactopyranoside. | | | | | | | | | |
| *Others: glucose fermentation, indole production, hydrolysis of esculin, hydrolysis of gelatin, and arginine dihydrolase. ^{(d} | | | | | | | | | |
| **Others: N-acetyl-D-glucosamine, m-hydroxy-benzoate, glycogen, phenyl acetate. The following carbon and energy sources were not utilized by any species or strains: N-acetylglucosamine, adipic acid, L-arabinose, capric acid, L- fucose, 2-keto-gluconate, 5-keto-gluconate, (D)glucose, (myo) inositol, itaconic acid, maltose, D-mannitol, D-melibiose, (L) rhamnose, D- ribose, (D) salicin, D-sorbitol, sucrose. The type of substrate used by Bowman et., al. (1996) is indicated in parenthesis. The type of optical isomer is indicated in parentheses in the Table. Frequencies of positive strains in the columns of *Psychrobacter pacificensis* in Table 2 above : + = 100%; +v = 67%; -v = 33%; and - = 0%. Frequencies of positive strains in the columns of other *Psychrobacter* species in Table 2 above: + = 100-90%; v+ = 89-11%; and v- = 10-0%. w: weak response. Strain NIBH P2K6 was defined as the reference strain of *Psychrobacter pacificensis.* | | | | | | | | | |

**Table 3**

| Fatty Acid Composition and Major Quinone Type of *Psychrobacter pacificensis* | | | | | |
|---|---|---|---|---|---|
| Composition | *Psychrobacter pacificensis* | | | | *Psychrobacter immobilis* |
| | NIBH strain no. | | | Average content | ATCC 43116 |
| | P2J3 | P2K6* | P2K18 | | |
| Fatty acid: | | | | | |
| 10:0 | 1.3 | Tr | 1.2 | 0.8 (0.7) | 0.9 |
| 11:0 | 0.1 | Tr | 0.2 | 0.1 (0.1) | Tr |
| 12:0 | 2.2 | 0.8 | 2.3 | 1.8 (0.8) | Tr |
| 14:0 | 0.7 | 0.6 | 0.5 | 0.6 (0.1) | 0.3 |
| 14:1 (X1) | 0.1 | 0.2 | Tr | 0.1 (0.1) | 0.1 |
| 15:0 | 0.4 | Tr | 0.4 | 0.3 (0.2) | 0.2 |
| 16:0 | 7.3 | 8.7 | 6.5 | 7.5 (1.1) | 4.3 |
| 16:1 (w7c) | 9.7 | 15.8 | 6.6 | 10.7 (4.7) | 3.8 |
| 16:1 (X2) | 0.4 | 0.4 | 0.3 | 0.4 (0.1) | 0.4 |
| 17:0 | 2.7 | 5.6 | 4.8 | 4.4 (1.5) | 4.2 |
| 117:0 | 0.6 | Tr | 0.4 | 0.3 (0.3) | Tr |
| 17:1 (X3) | 5.1 | 1.5 | 5.1 | 3.9 (2.1) | 6.8 |
| 18:0 | 9.4 | 5.6 | 13.1 | 9.4 (3.8) | 8.0 |
| 18:1 (w7c) | 1.2 | 0.8 | 0.7 | 0.9 (0.3) | 0.8 |
| 18:1 (w9c) | 50.1 | 52.8 | 50.9 | 51.3 (1.4) | 63.1 |
| 18:2 | 3.4 | 2.4 | 1.8 | 2.5 (0.8) | 3.5 |
| 19:0 | 0.4 | 0.4 | 0.6 | 0.5 (0.1) | 0.9 |
| 20:0 | Tr | Tr | Tr | Tr | 0.1 |
| Total | 95.1 | 95.6 | 95.4 | 95.5 | 97.4 |
| Total unsaturated | 70.0 | 73.9 | 65.4 | 69.8 | 75.0 |

| Hydroxy fatty acid: | | | | | |
|---|---|---|---|---|---|
| 3-OH 12:0 | 4.1 | 3.6 | 3.9 | 3.9 (0.3) | 2.2 |
| 3-OH 14:0 | 0.8 | 0.8 | 0.7 | 0.8 (0.1) | 0.4 |
| Total | 4.9 | 4.4 | 4.6 | 4.6 (0.3) | 2.6 |
| Major quinone type | Q-8 | Q-8 | Q-8 | Q-8 | Q-8 |
| X1-3: Accurate location of double bond has not been determined. Tr: trivial (<0.1 %)_{°} Numbers in parentheses indicate standard deviations (n=3)_{°} *: Reference strain. | | | | | |

*Psychrobacter pacificensis* is an aerobic, gram-negative, nonmotile, colorless, non-sporulating, and oxidase-positive coccobacillus of 1.0-1.5 µm long × about 1 µm wide. *Psychrobacter pacificensis* strains produce a number of fimbriae as extracellular organs but not flagella. Off-white colored, round, convex colonies with entire margins form on an agar plate containing polypeptone and yeast extract. No fluorescent color is formed. For optimum growth, seawater, or about 3% NaCl, may be required though most of the strains will not grow in the presence of 0%, 8% or higher NaCl. It takes 1-2 weeks for those strains to reach the stationary phase at 4°C, though these strains exhibit growth rates at 4°C comparable to their growth yields at 20°C. Optimum growth may be obtained at about 25°C, and the critical growth temperature is 38°C. Acids may be aerobically produced from glucose, xylose and arabinose. These strains are urease-activity-positive but phenylalanine deaminase- and tryptamine deaminase- negative. This species is negative in biochemical tests for glucose fermentation, indole production, hydrolysis of esculin, hydrolysis of gelatin and arginine dihydrase. This species utilizes L-histidine and DL-malic acid as sole carbon and energy sources. Some strains utilize acetic acid, L-alanine, 3-hydroxy-butyrate, lactic acid, malonic acid and suberic acid while none of these strains utilize p-hydroxy-benzoate, citric acid, gluconic acid, propionic acid, L-serine or n-valeric acid. 18:1(w9c) is the major fatty acid and Q8 is the major quinone. DNA G+C content was 43-44 mol% as determined by HPLC. NIBH P2K6 isolated from seawater collected from the Japan Trench in the offing of Hachijo island, Japan at a depth of 6,000m at 4°C, is used as a reference strain. This strain was deposited at the Institute for Fermentation, Osaka (IFO 16270). *Psychrobacter pacificensis* strain NIBH P2K6 was deposited (IFO 16270) at the Fermentation Research Institute, 1-1-3, Higashi, Tsukuba-shi, Ibaraki-ken, Japan) dated May 21, 1999 (Accession No.: FERM BP-7106).

*Psychrobacter pacificensis* is a newly-discovered species (Maruyama et al., Phylogenetic analysis of psychrophilic bacteria isolated from the Japan Trench, including a description of the deep-sea species *Psychrobacter pacificensis* sp. nov., Inter. J. Syst. Evol. Microbiol., 50 (2000) 835-846) and a number of its analogs have been isolated from seawater collected near the South Pole (Bowman et al., (1996) Int. J. Syst. Bacteriol. 46:841-848, Bowman et al., (1997) System. Appl. Microbiol. 20:209-215). Therefore, it will be appreciated that it is useful as an indicator organism with regard to the global circulation of deep-layer sea water. As one characteristic aspect of the global circulation of deep-layer sea water, it is known that deep-layer sea water in the Pacific Ocean flows steadily from the South Pole to the Japan Trench (Stommel, H.(1958) Deep-Sea Res. 5:80-82).

The newly-discovered bacterium species according to the present invention *Psychrobacter pacificensis* is, as described above, a novel indicator organism of deep-layer seawaters and abyssal zones, and thus may be useful in deep-layer seawater/abyssal zone monitoring systems for a variety of purposes. Recently, deep-layer seawater is intensively researched as a recyclable bulk resource which has resource values of containing plenty of nutrients, keeping at low temperature and being clean (Resource value and use of deep-layer seawater, Kaiyo Monthly, Vol.26, No.3, 133-138, 1994). In the marine products industry, for example, culture of food plankton or seaweed, and feeding of cold-water marine animals or deep-sea organism are being studied. On the other hand, in the energy industry, application of deep-sea water to water temperature control, refrigeration and fresh water production is being tested. A organism monitoring technique for assessing deep-layer seawater is essential for utilization of such deep-layer seawater as a resource. Further, *Psychrobacter pacificensis* is a useful indicator organism in: monitoring of deep-layer seawater associated with disposal of industrial waste into abyssal zones; monitoring of abyssal microorganisms associated with utilization of deep-sea fish; research on the circulation of deep-layer seawater on a global scale; or monitoring the upwelling zone of deep-sea water.

Further, there is the possibility that *Psychrobacter pacificensis* may be an microorganism that produces useful materials, and can be used in the production by microorganism of, for example, chitinase (Bioindustry, the March issue, 5-12, 1998, CMC), cold-lipase or protease which is active at low temperatures (useful as a detergent) (Higashihara, Takanori "Marine microorganism and biotechnology", Edited by Shimizu, Ushio, Giho-do Shuppan, pp. 51-67, 1991) (Ohgiya et al., In: Biotechnological Application of Cold-adapted Organisms. Edited by R. Margesin and F. Schinner, Springer, pp. 17-34, 1999), or lipids which contain useful fatty acid or acids such as EPA, DHA or the like (Appl. Environ. Microbiol., 51, 730-737, 1986).

16S rDNA having the base sequence represented by SEQ ID NO:1 can be obtained from *Psychrobacter pacificensis* NIBH P2K6. Particularly, genomic DNA may be extracted from *Psychrobacter pacificensis* NIBH P2K6 cells by any standard method, and 16S rDNA may be then amplified by PCR using appropriate primers (Lane, D.L. (1991) 16S/23S rRNA sequencing. In Nucleic Acid Techniques in Bacterial Systematics, pp.115-175. Edited by E. Stackebrandt, M.Goodfellow. West Sussex: John Wiley & Sons). An excess amount of primer and dNTP may be removed from the resulting PCR products and then the purified PCR products can be sequenced directly by cycle sequencing process using appropriate primers. The determined sequence is shown in SEQ ID NO:1.

Based on the base sequence information of 16S rRNA gene from *Psychrobacter pacificensis* strain NIBH P2K6, a certain region in the base sequence specific to the bacterium may be extracted and a DNA probe may be prepared which enables molecular or cell level detection of the bacterium. Regions specific to the bacterium include one comprising nucleotide Nos. 458 to 476 of the nucleotide sequence of SEQ ID NO: 1 (or nucleotide Nos. 469-487 in the corresponding *E*. *coli.* sequence. A DNA probe of 10-50bp in size, preferably 15-25bp in size, which corresponds to this region may be prepared. One preferable example may be a probe having the following base sequence:

• 5' TAATGTCATCGTCCCCGGG 3' (SEQ ID NO:2)

A probe can be synthesized by phosphoramide process (Beacage and Carruthers, Tetrahedron Lett. 22:1859-1862 (1981)) or triester method (Matteucci et al., J. Am. Chem. Soc. 103:3185 (1981)). Alternatively, a probe may be synthesized in an automatic synthesizer.

Further, a probe may be labeled with an isotope, a fluorescent label, DIG (digoxigenin) or the like. Examples of a label may include fluorescent colorants such as Cy5 (indodicarbocyanine), TRITC (tetramethyl rhodamine isothiocyanate) or FITC (fluorescein isothiocyanate), and haptens such as DIG (digoxigenin).

A variety of processes for hybridization (e.g., Southern Blotting, Northern Blotting, Colony Hybridization or in-situ hybridization such as FISH; Fluorescence In Situ Hybridization)
using a probe according to the present invention can be used to detect or identify *Psychrobacter pacificensts*, *Psychrobacter glacincola* and analogs thereof. Analogs of *Psychrobacter pacificensis* and *Psychrobacter glacincola* may include those contained in a database which do not have well-grounded identifications, *Psychrobacter glacincola* (AFO25555, PGU85879, PGU85878, PGU85877, PGU85876), *Psychrobacter immobilis* (PIU85880), *Psychrobacter* sp. (PSU85874) and the like. A bacterium belonging to *Psychrobacter pacificensis* can also be species-specifically detected or identified by the above-described processes.

One example of a process for detecting or identifying *Psychrobacter pacificensis* by using a probe having the nucleotide sequence of SEQ ID NO:2 will be described below.

A microorganism sample fixed by using, for example, paraformaldehyde is applied to a glass slide containing an organic film such as gelatin film formed thereon to immobilize the microorganism cells on the organic film. After dehydrating with ethanol or drying the microorganism cells at room temperature overnight, genomic DNA and RNA from the microorganism are allowed to hybridize to the DNA probe, and free- or incompletely bound-DNA probes are removed by washing. Any conditions suitable for hybridization can be used though relatively moderate conditions which permit 1-2 mismatches, preferably at 40-46°C for 4 hours or more without formamide, may be used for detecting or identifying *Psychrobacter pacificensis*, *Psychrobacter glacincola* and analogs thereof, while stringent conditions which will not allow any mismatch, preferably at 44-48°C for 4 hours or more in the presence of approximately 30-40% formamide, may be used for species-specific detection or identification of a microorganism belonging to *Psychrobacter pacificensis.* Microorganism cells are observed according to a conventional fluorescence microscopy procedure to detect fluorescence of the fluorescence-labeled DNA probe which has complementarily hybridized to nucleic acids from the subject microorganism. As a control, the same experiment may be performed using a non-specific DNA probe and a different microorganism belonging to another species. When a microorganism for which the DNA probe is targeted is used, it can be detected or identified since nucleic acids in cells will complementarily hybridize to the DNA probe thereby causing the cells to emit fluorescence.

The probe according to the present invention will not only enable species-specific detection of *Psychrobacter pacificensis*, *Psychrobacter glacincola* and analogs thereof, as well as of a microorganism belonging to *Psychrobacter pacificensis*, but also will be very useful to enable a quicker and more accurate detection.

The present invention will be described in detail with reference to the following examples which are included for purposes of illustration only and are not intended to limit the scope of the invention.

### Example 1

### Isolating Psychrobacter pacificensis strains and sequencing 16S rRNA gene

Among 67 strains isolated from surface waters and the abyssal environment of the Japan Trench, 16 strains in total were selected. Eleven of the 16 strains were tentatively identified to be bacteria similar to *Moraxellaceae.* Different agar media such as 1/2 TZ based on an artificial seawater containing polypeptone and yeast extract (Maruyama, A et al., (1993) J. Oceanogr. 49, 353-367), Marine Agar (Difco; Detroit, MI, USA), Nitrient Agar (Difco) and the like were used to purify the strains. Each strain was incubated at 20°C and collected to obtain genomic DNA. A 1/2 TZ semi-solid agar medium containing 0.3% agar was used for storage at 4°C. Dry cells enclosed in a glass test tube was also stored at 4°C, for a long period of time.

16S rRNA gene from those strains similar to *Moraxellaceae* isolated from the deep-sea water was determined by direct sequencing described below. Particularly, cells were collected by centrifugation, washed and resuspended in a TE buffer (10 mM Tris-HCl, 1 mM EDTA; pH 8.0). Cetyltrimethylammonium bromide (CTAB), phenol and chloroform/isoamyl alcohol (Murray, M.G. et al., (1980) Nucleic Acids Res 8, 4321-4325) were used to extract genomic DNAs by standard methods. In order to obtain almost-complete-16S rRNA gene, primers 27f and 1525r (Lane, D.L. (1991) 16S/23S rRNA sequencing. In Nucleic Acid Techniques in Bacterial Systematics, pp.115-175. Edited by E. Stackebrandt, M.Goodfellow. West Sussex: John Wiley & Sons) and the PCR cycle described in Maruyama, A. et al., (1997) Mar. Biol 128, 705-711 were used to perform PCR amplification with Gene Amp PCR 9600 (Perkin-Elmer, Norwalk, Conn., USA). SUPREC-02 (Takara Shuzo Co., Ltd.) was used to remove an excess amount of primer and dNTP from the resultant PCR amplification products. An automatic DNA sequencer (ALFred; Pharmacia LKB, Sweden) was used to directly sequence the purified PCR products by cycle sequencing process according to the manufacturer's instruction using appropriate forward and reverse primers (Lane, D.L. (1991), *supra).* Particularly, the above-described primers were, *E*. *coli* numbering, 342r, 359f (5'-TCC TAC GGG AGG CAG CAG TG (SEQ ID NO:3); 20-mer), 519r, 803r (5'-CAT CGT TTA CGG CGT GGA C (SEQ ID NO:4); 19-mer), 821f (5'-GTC CAC GCC GTA AAC GAT G (SEQ ID NO:5); 19-mer), 1104r (5'-TTG CGC TCG TTG CGG GAC (SEQ ID NO:6); 18-mer), and 1111f (5'-GTC CCG CAA CGA GCG CAA (SEQ ID NO:7); 18-mer). Both strands of each fragment of 16S rDNA region were sequenced and ligated by using GENETYX software (version 8; Software Development Co., Ltd.). Except for the abyssal strains similar to *Moraxellaceae* of which 16S rDNA had been analyzed as mentioned above, 16S rDNA was extracted from other strains including the standard species *Moraxella lacunata* (ATCC 17967) as previously described in Maruyama, A. et al., (1997) *(supra),* amplified and subcloned. A multi-alignment program in CLUSTAL W (version 1.71; 44) was used to align these sequences. CLUSTAL W profile alignment option was used to align the sequences determined by the present inventors to known aligned sequences obtained from the rRNA www server (http://rrna,uia,ac,be/;45) of University of Antwerp. All locations which include a gap or gaps (i.e., undetermined or not-well-grounded sequences) were removed from the aligned data matrix. The nucleotide sequence of 16S rRNA gene from *Psychrobacter pacificensis* NIBH P2K6 is shown in SEQ ID NO:1.

### Example 2

### Phylogenetic characteristics of Psychrobacter pacificensis

As described in Example 1, *Psychrobacter pacificensis* is a heterotrophic microorganism which appears predominantly under cold culture conditions of 1 atm, 4°C, from the seawater of the Japan Trench in the offing of Hachijo island, Japan at a depth of 6,000 meters. Six strains of *Psychrobacter pacificensis*, NIBH P2J2, NIBH P2J3, NIBH P2J13, NIBH P2K6, NIBH P2K17 and NIBH P2K18 have been isolated by the present inventors.

Among these strains, the phylogenetic characteristics of NIBH P2J3, NIBH P2K6 and NIBH P2K18 were examined according to conventional methods. The results are shown in Tables 4-6 below. Tables 4-6 also include other strains isolated simultaneously with, and analogs of, the above-described strains together with their phylogenetic characteristics.

**Table 4**

| Motility and Extracellular Organ of Psychrotrophic Bacteria Isolated from Surface and Deep Seawaters of the Japan Trench | | | | |
|---|---|---|---|---|
| Strain | Motility test¹ (microscopic) | Motility test² (agar plate) | Extracellular organ³ | Phylogenetic location |
| Surface seawater | | | | |
| P1H8 | - | - | Flagella* | *Halomonadaceae* |
| P1H13 | - | - | None | *Halomonadaceae* |
| P1H14 | - | - | None | *Halomonadaceae* |
| P1H22 | + | + | Flagella | *Halomonadaceae* |
| P1H25 | + | + | Flagella | *Halomonadaceae* |
| | | | | |

| Deep seawater | | | | |
|---|---|---|---|---|
| P2J2 | -/w | - | Fimbriae | *Moraxellaceae* |
| P2J3 | -/w | - | Fimbriae | *Moraxellaceae* |
| P2J13 | -/w | - | Fimbriae | *Moraxellaceae* |
| P2K6 | -/w | - | Fimbriae | *Moraxellaceae* |
| P2K17 | -/w | - | Fimbriae | *Moraxellaceae* |
| P2K18 | -/w | - | Fimbriae | *Moraxellaceae* |

| | | | | |
|---|---|---|---|---|
| 1: By optical microscopy using Nomarski optics. 2: On a semi-solid agar medium with nutrient gradient. 3: By electron microscopy:- (negative); Flagella* (frequent adhesion of flagella was observed); w (weak twitch). | | | | |

**Table 5**

| Characteristics of Phenotypes and GC Contents of *Psychrobacter pacificensis* strains and Analogs thereof | | | | | | | | | |
|---|---|---|---|---|---|---|---|---|---|
| Characteristics^{(a} | *Psychrobacter pacificensis* NIBH strain no. | | | | *Psychrobacter immobilis* ^{(b} | *Psychrobacter urativorans* ^{(b} | *Psychrobacter frigidicola* ^{(b} | *Psychrobacter phenylpyruvicus* | *Psychrobacter glacincola* |
| | P2J3 | P2K6 | P2K18 | Summary | (Phenon 1) | (Phenon 2) | (Phenon 3) | ACAM 535^{(b} | ACAM 483^{a)} |
| Urease activity | + | + | + | + | v+ | v+ | - | + | v- |
| Phenylalanine deaminase | - | - | - | - | + | - | + | + | - |
| Tryptophan deaminase | - | - | - | - | v- | - | + | - | -- |
| Nitrate reduction | - | - | - | - | v- | v- | - | - | v+ |

| Growth in NaCl (%): | | | | | | | | | |
|---|---|---|---|---|---|---|---|---|---|
| 0 | w | - | - | - | + | + | + | ? | + |
| 1 | + | - | - | -v | + | + | + | + | + |
| 3 | + | + | + | + | + | + | + | + | + |
| 5 | + | + | + | + | + | + | + | + | + |
| 8 | - | - | - | - | + | + | + | + | + |

| Growth at (°C): | | | | | | | | | |
|---|---|---|---|---|---|---|---|---|---|
| 30 | + | + | + | + | + | - | - | + | - |
| 35 | + | w | + | + | v+ | - | - | + | - |
| 40 | - | - | - | - | | | | | |

| Acid production from: | | | | | | | | | |
|---|---|---|---|---|---|---|---|---|---|
| Glucose | + | w | + | + | | | | | - |
| Xylose | + | w | + | + | + | - | - | - | - |
| Arabinose | + | + | + | + | + | - | - | - | - |
| *Others^{(d} | - | - | - | - | - | - | - | - | - |
| PNPG test^{(d} | - | - | - | - | | | | | |

| Use as sole carbon and energy sources: | | | | | | | | | |
|---|---|---|---|---|---|---|---|---|---|
| Acetate | + | - | - | -v | + | v+ | + | + | + |
| L-alanine | + | - | - | -v | + | - | - | + | v+ |
| p-hydroxy-benzoate | - | - | - | - | - | v- | - | - | 7 |
| 3-hydroxy-butytate | + | - | + | +v | + | + | - | + | v+ |
| Citrate | - | - | - | - | v- | - | - | + | v+ |
| Gluconate^{(d} | - | - | - | - | v- | - | - | - | - |
| L-histidine | + | + | + | + | + | - | - | - | v+ |
| Lactate | + | - | + | +v | +(DL) | v+ (DL) | - (DL) | + (DL) | v+(DL) |
| DL-malate^{(d} | + | + | + | + | +(L) | v+(L) | +(L) | + (L) | -(L) |
| Malonate | - | + | - | -v | - | - | - | - | - |
| Propionate | - | - | - | - | v+ | - | - | + | + |
| L-serine | - | - | - | - | - | v- | - | - | - |
| Suberate | + | - | - | -v | v- | - | + | - | ? |
| n-valerate | - | - | - | - | + | v+ | + | + | + |
| **Others | - | - | - | - | | | | | |
| DNA G+C content (mol %) | 44 | 44 | 45 | 44-45 | 44-47 | 44-46 | 41-12 | 43 | 43-14 |

| | | | | | | | | | |
|---|---|---|---|---|---|---|---|---|---|
| a) All of the species and strains were proved to be positive for oxidase, catalase, culture at 4-15°C, resistance against 6.5% NaCl, as well as for the use of L-proline as sole carbon and energy sources. b) Data from Bowman et al., (1996) Int. J. Syst. Bacteriol. 46:841-848. c) Data from Bowman et al., (1997) System. Appl. Microbiol. 20:209-215. d) Determined by API 20 NE test. Compound availability was estimated using API ID 32 GN test. PNPG is a test for β-galactosidase using para-nitrophenyl-( β)-D-galactopyranoside. | | | | | | | | | |
| *Others: glucose fermentation, indole production, hydrolysis of esculin, hydrolysis of gelatin, and arginine dihydrolase. ^{(d} | | | | | | | | | |
| **Others: N-acetyl-D-glucosamine, m-hydroxy-benzoate, glycogen, phenyl acetate. The following carbon and energy sources were not utilized by any species or strains: N-acetylglucosamine, adipic acid, L-arabinose, capric acid, L- fucose, 2-keto-gluconate, 5-keto-gluconate, (D)glucose, (myo) inositol, itaconic acid, maltose, D-mannitol, D-melibiose, (L) rhamnose, D- ribose, (D) salicin, D-sorbitol, sucrose. The type of the substrate used by Bowman et., al. (1996) is indicated in parathesis. The type of optical isomer is indicated in parentheses in the Table. Frequencies of positive strains in the columns of *Psychrobacter pacificensis* in Table 2 above : + = 100%; +v = 67%; -v = 33%; and - = 0%. Frequencies of positive strains in the columns of other *Psychrobacter* species in Table 2 above: + = 100-90%; v+ = 89-11%; and v- = 10-0%. w: weak response. Strain NIBH P2K6 was defined as the reference strain of *Psychrobacter pacificensis*. | | | | | | | | | |

**Table 6**

| Fatty Acid Composition and Major Quinone Type of *Psychrobacter pacificensis* | | | | | |
|---|---|---|---|---|---|
| Composition | *Psychrobacter pacificensis* | | | | *Psychrobacter immobilis* |
| | NIBH strain no. | | | Average content | ATCC 43116 |
| | P2J3 | P2K6* | P2K18 | | |
| Fatty acid: | | | | | |
| 10:0 | 1.3 | Tr | 1.2 | 0.8 (0.7) | 0.9 |
| 11:0 | 0.1 | Tr | 0.2 | 0.1 (0.1) | Tr |
| 12:0 | 2.2 | 0.8 | 2.3 | 1.8 (0.8) | Tr |
| 14:0 | 0.7 | 0.6 | 0.5 | 0.6 (0.1) | 0.3 |
| 14:1 (X1) | 0.1 | 0.2 | Tr | 0.1 (0.1) | 0.1 |
| 15:0 | 0.4 | Tr | 0.4 | 0.3 (0.2) | 0.2 |
| 16:0 | 7.3 | 8.7 | 6.5 | 7.5 (1.1) | 4.3 |
| 16:1 (w7c) | 9.7 | 15.8 | 6.6 | 10.7 (4.7) | 3.8 |
| 16:1 (X2) | 0.4 | 0.4 | 0.3 | 0.4 (0.1) | 0.4 |
| 17:0 | 2.7 | 5.6 | 4.8 | 4.4 (1.5) | 4.2 |
| i17:0 | 0.6 | Tr | 0.4 | 0.3 (0.3) | Tr |
| 17:1 (X3) | 5.1 | 1.5 | 5.1 | 3.9 (2.1) | 6.8 |
| 18:0 | 9.4 | 5.6 | 13.1 | 9.4 (3.8) | 8.0 |
| 18:1 (w7c) | 1.2 | 0.8 | 0.7 | 0.9 (0.3) | 0.8 |
| 18:1 (w9c) | 50.1 | 52.8. | 50.9 | 51.3(1.4) | 63.1 |
| 18:2 | 3.4 | 2.4 | 1.8 | 2.5 (0.8) | 3.5 |
| 19:0 | 0.4 | 0.4 | 0.6 | 0.5 (0.1) | 0.9 |
| 20:0 | Tr | Tr | Tr | Tr | 0.1 |
| Total | 95.1 | 95.6 | 95.4 | 95.5 | 97.4 |
| Total unsaturated | 70.0 | 73.9 | 65.4 | 69.8 | 75.0 |

| Hydroxy fatty acid: | | | | | |
|---|---|---|---|---|---|
| 3-OH 12:0 | 4.1 | 3.6 | 3.9 | 3.9 (0.3) | 2.2 |
| 3-OH 14:0 | 0.8 | 0.8 | 0.7 | 0.8 (0.1) | 0.4 |
| Total | 4.9 | 4.4 | 4.6 | 4.6 (0.3) | 2.6 |
| Major quinone type | Q-8 | Q-8 | Q-8 | Q-8 | Q-8 |
| X1-3: Accurate location of double bond has not been determined. Tr: trivial (<0.1%). Numbers in parentheses indicate standard deviations (n=3). *: Reference strain. | | | | | |

*Psychrobacter pacificensis* is an aerobic, gram-negative, nonmotile, colorless, non-sporulating, and oxidase-positive coccobacillus of 1.0-1.5 µm long × about 1 µm wide. *Psychrobacter pacificensis* strains produce a number of fimbriae as the extracellular organs but not flagella. Off-white colored round convex colonies with entire margins are formed on an agar plate containing polypeptone and yeast extract. No fluorescent color is formed. For optimum growth, seawater or about 3% NaCl aqueous solution may be required though most of the strains will not grow in the presence of 0%, 8% or higher NaCl. It takes 1-2 weeks for those strains to reach the stationary phase at 4°C though these strains exhibit growth rates at 4°C comparable to their growth yields at 20°C. Optimum growth may be obtained at about 25°C and the critical growth temperature is 38 °C. Acids may be aerobically produced from glucose, xylose and arabinose. These strains are urease-activity-positive but phenylalanine deaminase- and tryptamine deaminase- negative. This species is negative in biochemical tests for glucose fermentation, indole production, hydrolysis of esculin, hydrolysis of gelatin and arginine dihydrase. This species utilizes L-histidine and DL-malic acid as sole carbon and energy sources. Some strains utilize acetic acid, L-alanine, 3-hydroxy-butyrate, lactic acid, malonic acid and suberic acid while none of these strains utilize p-hydroxy-benzoate, citric acid, gluconic acid, propionic acid, L-serine or n-valeric acid. 18:1(w9c) is the major fatty acid and Q8 is the major quinone. DNA G+C content was 43-44 mol% as determined by HPLC. NIBH P2K6 isolated from seawater collected from the Japan Trench in the offing of Hachijo island, Japan at a depth of 6,000m at 4°C is used as a reference strain. This strain has been deposited at the Institute for Fermentation, Osaka (IFO 16270). *Psychrobacter pacificensis* strain NIBH P2K6 has been deposited (IFO 16270) at the Fermentation Research Institute, 1-1-3, Higashi, Tsukuba-shi, Ibaraki-ken, Japan) dated May 21, 1999 (Accession No.: FERM BP-7106).

Although *Psychrobacter pacificensis* had initially been named *"Psychrobacter pacificus"* by the present inventors, it was later renamed *"Psychrobacter pacificensis"* and registered as a new species (Maruyama et al., Phylogenetic analysis of psychrophilic bacteria isolated from the Japan Trench, including a description of the deep-sea species *Psychrobacter pacificensis* sp. nov., Inter. J. Syst. Evol. Microbiol., 50 (2000) 835-846). Accordingly, it is internationally recognized that *Psychrobacter pacificensis* is a newly-discovered microorganism.

### Example 3

### Result of Database Search for Probe Prepared

A probe sequence having the nucleotide sequence of SEQ ID NO:2 (named "Psypac469-487") was searched in RDP-DB (database) under conditions that permit up to 2 mismatches in the sequence. There was no sequence except for the *Psychrobacter pacificensis* which matched to Psypac469-487 among the standard strains presently registered (Type-species) and those with well-grounded identification. However, in the database, Accession No. AF025555 (Pinhass et. al.; a partial sequence of 300bp; indicated by *Psychrobacter glacincola*) had 0 mismatches, U85874 (Bowmann et. al., Appl. Environ. Microbiol. 63, 3068-3078, 1997; indicated by *Psychrobacter* sp.), U85876, U85877, U85878 and U85879 (Bowmann et. al., Appl. Environ. Microbiol. 63, 3068-3078, 1997; indicated by *Psychrobacter glacincola*), U85880 (Bowmann et. al., Appl. Environ. Microbiol. 63, 3068-3078, 1997; indicated by *Psychrobacter immobilis*) had 1 mismatch, and U85875 (Bowmann et. al., Appl. Environ. Microbiol. 63, 3068-3078, 1997; indicated by *Psychrobacter* sp.), AF025579 (Pinnhassi et. al.; partial sequence of 442bp; indicated by *Psychrobacter* sp. Ant9) and AF025577 (Pinnhassi et. al.; partial sequence of 501bp; indicated by *Moraxella* sp. Ant7) had 2 mismatches. Identifications of those strains from which these sequences contained in the sequence data were derived were not well-grounded, and there remains a possibility that *Psychrobacter pacificensis* might be included among them. Further, since most of these nucleotide sequences homologous to that of the *Psychrobacter pacificensis* were derived from bacterial strains obtained in the Antarctic region, and as indicated, these may possibly be derived from *Psychrobacter glacincola,* which has been already proved to live in the Antarctic region or an analog thereof.

These results indicate that the base sequence of Psypac469-487 probe is complementary to the base sequence of *Psychrobacter pacificensis*, *Psychrobacter glacincola* and analogs thereof, in a DNA database that encompasses all of the existing strains, thus showing that the probe may be very useful for species-specific detection of the two species.

### Example 4

### Hybridization Test of Probe Prepared (1)

### (1) Preparation of microorganism sample

Microorganisms (genus, species and strain) used herein are listed in Table 7. *Psychrobacter pacificensis* strains were isolated as microorganisms viable at 4°C from a deep-sea water sample collected from the Japan Trench in the offing of Hachijo island, Japan at a depth of 6,000m, but they could not be found at all in surface water (Maruyama et al. Marine Biology 128, 705-711, 1997). *Bacillusmarinus* was cultured under an aerobic condition at 20°C using Marine Broth (Difco), and *Psychrobacter phenylpyruvicus* was cultured under an aerobic condition at 30°C using ATCC Culture Medium #4. The rest of the bacteria were cultured under aerobic conditions at 10-20°C using 1/2TZ liquid media (Maruyama et al., J. Oceanogr. 49, 353-367, 1993).

Cultured microorganisms were fixed at 4°C overnight using a final concentration of 3% paraformaldehyde. The paraformaldehyde was first dissolved in 3 × PBS (Phosphate Buffer Saline: 24g of NaCl, 0.6g of KCl, 0.72g of Na₂HPO₄, pH7.4) to a concentration of 15%, and then used in combination with samples (sample: paraformaldehyde=4:1).

### (2) Staining Sample with DNA Probe

The fixed microorganism sample was adsorbed on a Teflon-coated slide with a sample hole (diameter: 11mm) formed therein to which gelatin had previously been applied, and ethanol-dehydrated or dried at room temperature overnight. Next, 50 µl of a hybridization solution (0.9M NaCl, 50mM sodium phosphate buffer (pH7.0), 5mM EDTA, 0.5% SDS, Denhardt solution (final × 1), 1.0mg/ml Poly(A)) was added to the sample hole. The glass slide prepared as described above was left to stand in a 50ml conical tube, loaded with a small amount of 3 × PBS to prevent drying, at 42-45°C for 30 minutes for prehybridization.

Oligonucleotide DNA probes having the 5' end thereof fluorescence-labeled with Cy5, TRITC (tetramethyl rhodamine isothiocyanate) or FITC (fluorescein isothiocyanate) were prepared and each added to the above-described hybridization solution at an amount of 1ng probe/µl solution, and hybridization was performed at 42-45°C for 4.5 hours. Unreacted oligonucleotide probe DNA was removed by washing the glass slide with wash solution (0.9M NaCl, 0.5mM sodium phosphate, 0.1% SDS, pH=7.0) at 44-45°C for 30 minutes.

Oligonucleotide DNA probes used herein were Psypac469-487, a common probe among Domain Bacteria (previously Eubacteria) Eub338-355 (5'-GCTGCCTCCCGTAGGAGT (SEQ ID NO: 8), and a control probe, Cont (5'-GTGCCAGCAGCCGCGG (SEQ ID NO:9)).

### (3) Staining Sample DNA

After completion of hybridization, DAPI solution (final concentration of 5 µg/ml) was added to the glass slide and left to stand at room temperature for 10 minutes to stain the DNAs present in the microorganism cells. After completion of the reaction, the glass slide was immersed in and washed with pure water for 15 minutes and then dried at room temperature.

### (4) Observation of Sample by Fluorescence Microscopy

An anti-color-degradation agent such as DABCO (diazabicyclooptane) solution (1 g/100 ml (10 ml PBS+90 ml Glycerol)) was added to the microorganism sample dried on the glass slide which was then covered with a cover glass and observed through a fluorescence microscope under oil immersion condition. Optionally, a fluorescent image for each colorant was captured by a cold CCD camera attached to the fluorescence microscope, and then analyzed. The results are shown in Table 7 below.

**Table 7**

| Results of Utility Test of Probe by Fluorescence Microscopy According to FISH | | | | |
|---|---|---|---|---|
| Strain | Fluorescence-labeled DNA probe | | | |
| | Control | Psypac 469-487 | Euba 338-355 | DAPI Staining |
| *Psychrobacter pacificensis* NIBH P2J2 | × | ○ | ○ | ○ |
| *P*. *pacificensis* NIBH P2J3 | × | ○ | ○ | ○ |
| *P*. *pacificensis* NIBH P2J13 | × | ○ | ○ | ○ |
| *P*. *pacificensis* NIBH P2K6 (=IFO 16270) | × | ○ | ○ | ○ |
| *P. pacificensis* NIBH P2K18 | × | ○ | ○ | ○ |
| *Psychrobacter glacincola* ACAM 483* | × | ○ | ○ | ○ |
| *Psychrobacter frifidicola* ACAM 304 | × | × | ○ | ○ |
| *Psychrobacter immobilis* ATCC 43116 | × | × | ○ | ○ |
| *Psychrobacter urativorans* ATCC 15174 | × | × | ○ | ○ |
| *Psychrobacter phenylpyruvicus* ATCC 233 | × | × | ○ | ○ |
| *Pseudomonas aeruginosa* IFO 12689 | × | × | ○ | ○ |
| *Vibrio parahaemolyticus* IFO 12711 | × | × | ○ | ○ |
| *Bacillus marinus* ATCC 29841 | × | × | ○ | ○ |

| | | | | |
|---|---|---|---|---|
| * Registered as *P. endoglaciecola* in the DNA database | | | | |

These results show that the probes prepared herein can species-specifically bind to a microorganism belonging to *Psychrobacter pacificensis* or *Psychrobacter glacincola* but not to microorganisms belonging to any other genera, by *in situ* hybridization (also referred to as "whole cell hybridization") using relatively moderate conditions which permit 1-2 mismatches.

### Example 5

### Hybridization Test of Probe Prepared (1)

### (1) Preparation of Microorganism Sample

Microorganism (genus, species and strain) used herein are listed in Table 8. *Psychrobacter pacificensis* strains were isolated as microorganisms viable at 4°C from the deep-sea water sample collected from the Japan Trench in the offing of Hachijo island, Japan at a depth of 6,000m, but they could not be found in the surface water at all (Maruyama et al. Marine Biology 128, 705-711, 1997). *Bacillus marinus* was cultured under an aerobic condition at 20°C using Marine Broth (Difco), and *Psychrobacter phenylpyruvicus* was cultured under an aerobic condition at 30°C using ATCC Culture Medium #4. The rest of the bacteria were cultured under aerobic conditions at 10-20°C using 1/2TZ liquid media (Maruyama et al., J. Oceanogr. 49, 353-367, 1993).

Cultured microorganisms were fixed at 4°C overnight using a final concentration of 3% paraformaldehyde. The paraformaldehyde was first dissolved in 3 × PBS (Phosphate Buffered Saline: 24g of NaCl, 0.6g of KCl, 0.72g of Na₂HPO₄, pH7.4) to a concentration of 15%, and then used in combination with samples (sample: paraformaldehyde=4:1).

### (2) Staining Sample with DNA Probe

The fixed microorganism sample was adsorbed on a Teflon-coated slide with a sample hole (diameter: 11mm) formed therein to which gelatin had previously been applied, and ethanol-dehydrated or dried at room temperature overnight. Next, 50 µl of a hybridization solution (0.9M NaCl, 50mM sodium phosphate buffer (pH7.0), 5mM EDTA, 0.5% SDS, Denhardt solution (final × 1), 1.0mg/ml Poly(A)) was added to the sample hole. The glass slide prepared as described above was left to stand in a 50ml conical tube loaded with a small amount of 3 × PBS to prevent drying, at 46°C for 30 minutes for prehybridization.

Oligonucleotide DNA probes having the 5' end thereof fluorescence-labeled with Cy5, TRITC (tetramethyl rhodamine isothiocyanate) or PITC (fluorescein isothiocyanate) were prepared and each added to the above-described hybridization solution at an amount of 1ng probe/µl solution. Hybridization was performed for each oligonucleotide DNA probe for 4.5 hours under the optimum condition (described below) for the probe. Unreacted oligonucleotide probe DNA were then removed by washing the glass slide with wash solution (0.9M NaCl, 0.5mM sodium phosphate, 0.1% SDS, pH=7.0) at 44°C for 30 minutes.

Oligonucleotide DNA probes and optimal hybridization conditions (within parentheses) used were as follows: Psypac469-487 (in 35% formamide solution at 46°C); a common probe among the Domain Bacteria (previously Eubacteria) Euba 338-355 (5'-GCTGCCTCCCGTAGGAGT: SEQ ID NO:8) (in 20% formamide solution at 46°C); and a universal probe Univ1390-1407 (5'-GACGGGCGGTGTGTACAA: SEQ ID NO:10) (in 0% formamide solution at 42°C)) (Maruyama and Sunamura, Applied and Environmental Microbiology, 66, 2211-2215, 2000). As a control, Cont (5'-GTGCCAGCAGCCGCGG: SEQ ID NO:9) (in 0% formamide solution at 42°C) was used to determine whether any non-specific adsorption occurred.

### (3) Staining Sample DNA

After completion of hybridization, DAPI solution (final concentration of 5 µg/ml) was added to the glass slide and left to stand at room temperature for 10 minutes to stain the DNAs present in the microorganism cells. After completion of the reaction, the glass slide was immersed in and washed with pure water for 15 minutes and then dried at room temperature.

### (4) Observation of Sample by Fluorescence Microscopy

An anti-color-degradation agent such as DABCO (diazabicyclooctane) solution (1 g/100 ml (10 ml PBS+90 ml Glycerol)) was added to the microorganism sample dried on the glass slide which was then covered with a cover glass and observed through a fluorescence microscope under oil immersion condition. Optionally, a fluorescent image for each colorant was captured by a cold CCD camera attached to the fluorescence microscope, and then analyzed. The results are shown in Table 8 below.

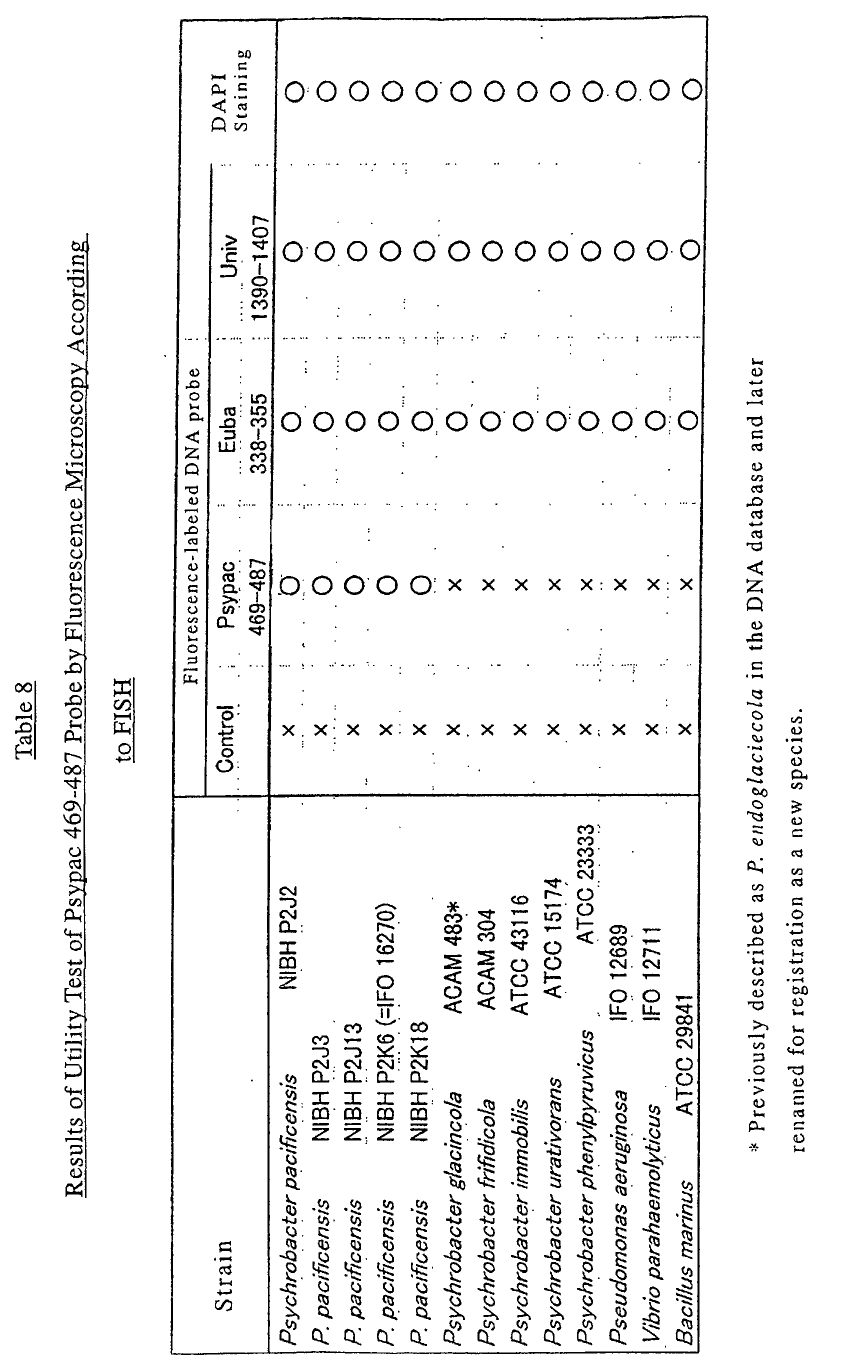

These results show that the probe prepared can specifically bind only to a microorganism belonging to *Psychrobacter pacificensis* but not to others, by *in situ* hybridization (also referred to as "whole cell hybridization") using stringent conditions which will not permit any mismatch.

It should be noted that all the publications, patents and patent applications cited herein are entirely incorporated herein by reference.

### Industrial Applicability

Use of an oligonucleotide probe according to the present invention enables highly-sensitive and accurate, molecular- or cell-level detection of *Psychrobacter pacificensis,* which is an indicator organism useful in understanding the circulation of deep-layer sea water. Since the use of a great number of microorganism samples is required for analysis of the behavior of deep-sea water and the evaluation of effects, enormous labor and time were required to perform complicated separating and culturing procedures as well as to classify and identify the microorganisms on land in conventional culture methods. However, use of the DNA probe according to the present invention, which comprises a base sequence, the species-specificity of which has been confirmed in an existing database, can provide quicker and easier detection or identification of *Psychrobacter pacificensis*, *Psychrobacter glacincola* and analogs thereof, as well as a quicker and easier species-specific detection and identification of *Psychrobacter pacificensis*. Further, *Psychrobacter pacificensis,* the novel psychrotrophic bacterium according to the present invention, can provide a novel indicator organism of deep-sea water and zones.

## Claims

1. A 16S rDNA which has the base sequence of SEQ ID NO:1.

2. An oligonucleotide probe which comprises part of the base sequence of SEQ ID NO:1.

3. The oligonucleotide probe according to claim 2 wherein said part of the base sequence of SEQ ID NO:1 comprises the base sequence of SEQ ID NO:2.

4. The oligonucleotide probe according to claim 2 or 3 for detecting or identifying a bacterium selected from the group consisting of *Psychrobacter pacificensis, Psychrobacter glacincola* and analogs thereof.

5. A method for detecting or identifying a bacterium selected from the group consisting of *Psychrobacter pacificensis*, *Psychrobacter glacincola* and analogs thereof, using an oligonucleotide probe comprising part of the base sequence of SEQ ID NO: 1.

6. The oligonucleotide probe according to claim 2 or 3 for specifically detecting or identifying a bacterium belonging to *Psychrobacter pacificensis.*

7. A method for specifically detecting or identifying a bacterium belonging to *Psychrobacter pacificensis*, using an oligonucleotide probe comprising part of the base sequence of SEQ ID NO:1.

8. *Psychrobacter pacificensis*, which is aerobic, gram-negative, nonmotile, colorless, non-sporulating and oxidase-positive.

9. The bacterium belonging to *Psychrobacter pacificensis* according to claim 6 wherein the bacterium is *Psychrobacter pacificensis* NIBH P2K6 (FERM BP-7106).


SEQUENCE LISTING

<110> Secretary of Agency of Industrial Science and Technology

<120> Novel Psychrotrophic Bacterium and DNA Probe for Detecting
      The Bacterium

<130> PH-999-PCT

<150> JP 11-145342
<151> 1999-05-25

<150> PCT/JP00/02045
<151> 2000-03-30

<160> 10

<170> PatentIn Ver. 2.0

<210> 1
<211> 1526
<212> DNA
<213> Psychrobacter pacificensis

<220>
<221> rRNA
<222> (1)..(1526)

<400> 1

tttgatcatg gctccagatt gaacgactgg gcggcaggct taacacatgc aagtcgagcg 60
gaaacgatga tagcttgcta ttaggcgtcg agcngccgga cgggtgagta atacttagga 120
atctacctag tagtggggga tagctcgggg aaactcgaat taataccgca tacgtctacg 180
ggagaaagca ggggntcatt agaccttgcg ctattagatg agcctaagtc ggattagcta 240
gatggtgggg taaaggccta ccatggcgac gatctgtagc tggtctgaga ggatgatcag 300
ccacaccggg actgagacac ggcccggact ctacgggagg cagcagtggg gaatattgga 360
caatggnggg aaccctgatc cagccatgcc gcgtgtgtga agaaggcctt ttggttgtaa 420
agcactttaa gcagtgaaga agactcttcg gttaataccc ggggacgatg acattagctg 480
cagaataagc accggctaac tctgtgccag cagccgcggt aatacagagg gtgcaagcgt 540
taatcggaat tactgggcgt aaagcgagcg taggtggctt gataagtcag atgtgaaatc 600
cccgggctta acctgggaac tgcatctgaa actgttaggc tagagtaggt gagagggaag 660
tagaatttca ggtgtagcgg tgaaatgcgt agagatctga aggaataccg atggcgaagg 720
cagcttcctg gcatcatact gacactgagg ctcgaaagcg tgggtagcaa acaggattag 780
ataccctggt agtccacgcc gtaaacgatg tctactagtc gttgggtccc ttgaggactt 840
agtgacgcag ctaacgcaat aagtagaccg cctggggagt acggccgcaa ggttaaaact 900
caaatgaatt gacgggggcc cgcacaagcg gtggagcatg tggtttaatt cgatgcaacg 960
cgaagaacct tacctggtct tgacatacac agaatcttgt agagatacga gagtgccttc 1020
gggaattgtg atacaggtgc tgcatggctg tcgtcagctc gtgtcgtgag atgttgggtt 1080
aagtcccgca acgagcgcaa cccttgtcct tagttaccag cacttcgggt gggaactcta 1140
aggatactgc cagtgacaaa ctggaggaag gcggggacga cgtcaagtca tcatggccct 1200
tacgaccagg gctacacacg tgctacaatg gtaggtacag agggcagcta cacagcgatg 1260
tgatgcgaat ctcaaaaagc ctatcgtagt ccagattgga gtctgcaact cgactccatg 1320
aagtaggaat cgctagtaat cgcggatcag aatgccgcgg tgaatacgtt cccgggcctt 1380
gtacacaccg cccgtcacac catgggagtt gattgcacca gaagtggtta gcctaactta 1440
gtgagggcga tcaccacggt gtggtcgatg actggggtga agtcgtaaca aggtagccgt 1500
aggggaacct gcggctggat cacctc 1526

<210> 2
<211> 19

<212> DNA
<213> Artificial Sequence

<220>
<223> Description of Artificial Sequence:Synthetic DNA

<400> 2
taatgtcatc gtccccggg                                                    19

<210> 3
<211> 20
<212> DNA
<213> Artificial Sequence

<220>
<223> Description of Artificial Sequence:Synthetic DNA

<400> 3
tcctacggga ggcagcagtg                                                   20

<210> 4
<211> 19
<212> DNA
<213> Artificial Sequence

<220>
<223> Description of Artificial Sequence:Synthetic DNA

<400> 4

catcgtttac ggcgtggac 19

<210> 5
<211> 19
<212> DNA
<213> Artificial Sequence

<220>
<223> Description of Artificial Sequence:Synthetic DNA

<400> 5
gtccacgccg taaacgatg 19

<210> 6
<211> 18
<212> DNA
<213> Artificial Sequence

<220>
<223> Description of Artificial Sequence:Synthetic DNA

<400> 6
ttgcgctcgt tgcgggac 18

<210> 7
<211> 18
<212> DNA
<213> Artificial Sequence

<220>
<223> Description of Artificial Sequence:Synthetic DNA

<400> 7
gtcccgcaac gagcgcaa                                                    18

<210> 8
<211> 18
<212> DNA
<213> Artificial Sequence

<220>
<223> Description of Artificial Sequence:Synthetic DNA

<400> 8
gctgcctccc gtaggagt                                                    18

<210> 9
<211> 16
<212> DNA
<213> Artificial Sequence

<220>
<223> Description of Artificial Sequence:Synthetic DNA

<400> 9
gtgccagcag ccgcgg                                                      16

<210> 10

<211> 18
<212> DNA
<213> Artificial Sequence

<220>
<223> Description of Artificial Sequence:Synthetic DNA

<400> 10
gacgggcggt gtgtacaa                                                    18